# EUROPEAN PATENT SPECIFICATION

(11) **EP 3 751 264 B1**
(45) Date of publication and mention of the grant of the patent: **18.10.2023**
(21) Application number: 20176549.2
(22) Date of filing: 26.05.2020
(51) Int. Cl.: C01F 17/206, C01F 17/224, C01F 17/235, G01N 33/00, G01N 27/12

(54) **CARBON DIOXIDE GAS SENSOR**
KOHLENDIOXIDGASSENSOR
CAPTEUR DE GAZ DE DIOXYDE DE CARBONE

(30) Priority: 14.06.2019 JP 2019111312
(43) Date of publication of application: 16.12.2020
(73) Proprietor: Fuji Electric Co., Ltd., Kawasaki-shi, Kanagawa 210-9530 (JP)
(72) Inventor: SUZUKI, Takuya, Kanagawa, 210-9530 (JP); BARSAN, Nicolae, 72076 Tübingen (DE); WEIMAR, Udo, 72076 Tübingen (DE)
(74) Representative: Appelt, Christian W.

(56) References cited:
- JP-A- 2007 024 508
- JP-A- 2007 229 558
- US-A- 4 432 948
- US-A- 5 993 624
- US-A1- 2009 148 347
- US-A1- 2016 334 359
- MICHEL CARLOS R ET AL: "Gas sensing properties of Gd2O3microspheres prepared in aqueous media containing pectin", SENSORS AND ACTUATORS B: CHEMICAL, ELSEVIER BV, NL, vol. 177, 23 November 2012 (2012-11-23), pages 390-396, XP028964107, ISSN: 0925-4005, DOI: 10.1016/J.SNB.2012.11.018
- PYNGROPE D ET AL: "Synthesis, Characterization and Comparative Luminescence Studies of Rare-Earth-Doped Gd2O3Nanoparticles", JOURNAL OF MATERIALS ENGINEERING AND PERFORMANCE, ASM INTERNATIONAL, MATERIALS PARK, OH, US, vol. 27, no. 6, 20 April 2018 (2018-04-20) , pages 2754-2758, XP036525933, ISSN: 1059-9495, DOI: 10.1007/S11665-018-3367-X [retrieved on 2018-04-20]

## Description

### BACKGROUND OF THE INVENTION

### FIELD OF THE INVENTION

The present invention relates to a carbon dioxide gas sensor and to a method for producing a carbon dioxide gas sensor.

### DESCRIPTION OF RELATED ART

Detection of carbon dioxide gas (hereinafter, also referred to as CO₂ gas) is attracting attention not only in the field of environmental safety, such as management of buildings and parking places, but also in the fields of agriculture and food-related industries. In a current standard technology, detection of CO₂ gas is conducted using a Non Dispersive Infrared (NDIR) CO₂ gas sensor. However, NDIR is expensive and bulky, and thus, there are problems that it is difficult to install. Therefore, a high performance chemoresistive CO₂ gas sensor achieving low cost and having a simple structure has been desired.

As a promising chemoresistive material used for a CO₂ gas sensor, rare earth metal oxycarbonate (rare earth oxycarbonate) has been proposed (for example, see Non-Patent Literatures 1 to 4). Although there are families of rare earth metal oxycarbonates having different rare earth metals and different crystal polymorphism, it is reported that a monoclinic lanthanum dioxycarbonate (La₂O₂CO₃) is the most suitable material for a CO₂ gas sensor (Non-Patent Literature 2).

In addition, semiconductor materials consisting of tin oxide (SnO₂) particles coated with lanthanum oxide (La₂O₃) or gadolinium oxide (Gd₂O₃) are known to be able to serve as a CO₂ gas sensor (for example, see Patent Literature 1).

US 2009/0148347 A1 discloses a nano-crystalline composite-oxide thin film for an environmental gas sensor, an environmental gas sensor using the thin film, and a method of manufacturing the environmental gas sensor.

MICHEL CARLOS R ET AL, "Gas sensing properties of Gd2O3 microspheres prepared in aqueous media containing pectin", SENSORS AND ACTUATORS B: CHEMICAL, ELSEVIER BV, NL, (20121123), vol. 177, discloses Gd2O3 hollow microspheres.

### CITATION LIST

### PATENT LITERATURE

[Patent Literature 1] Japanese Patent Laid-Open No. 2017-106857

### NON-PATENT LITERATURE

[Non-Patent Literature 1] Chem. Mater. 21 (2009) 5375-5381
[Non-Patent Literature 2] Journal of Sensors, Volume 2017, Article ID 9591081, 6 pages
[Non-Patent Literature 3] PNAS December 29, 2015. 112 (52) 15803-15808
[Non-Patent Literature 4] Electrochimica Acta 127 (2014) 355-361

### SUMMARY OF THE INVENTION

The present invention relates to a carbon dioxide gas sensor and a method for producing a carbon dioxide gas sensor.

The inventors investigated chemoresistivity of rare earth metal compounds, and as a result, the inventors discovered oxides suitable for a gas sensing layer of a CO₂ gas sensor and a method of production thereof, and finally completed the present invention.

The present invention relates to a carbon dioxide gas sensor according to claim 1 and a method for producing carbon dioxide gas sensor according to claim 2.

In the above gas sensor, the rare earth oxide represented by Ln₂O₃ is at least one of Sm₂O₃, Eu₂O₃, Tb₂O₃, Dy₂O₃, Er₂O₃ or Yb₂O₃.

In the above gas sensor, the rare earth oxide comprises a rare earth oxide having a cubic crystal structure as a main component.

According to another aspect of the invention, the present invention relates to a method for producing a carbon dioxide gas sensor according to claim 2.

### Advantageous Effects of Invention

According to the present invention, a compact, high-performance, chemoresistive CO2 gas sensor comprising a gas sensing layer containing a rare earth oxide can be obtained. Also, according to the present invention, a method for producing a carbon dioxide gas sensor can be achieved.

### BRIEF DESCRIPTION OF THE DRAWINGS

FIG. 1 is a schematic view showing a cross-sectional structure of the gas sensor according to one aspect in an embodiment of the present invention.
FIG. 2 is a schematic view showing a cross-sectional structure of the gas sensor according to another aspect of the present invention.
FIGS. 3A to 3C show the properties for eight gas sensors each comprising one of gas sensing layers composed of different rare earth oxides investigated under the conditions of carbon dioxide in a concentration of 1000 ppm at 20°C and 50% RH and an operating temperature of 300°C. FIG. 3A is a graph showing evaluation results of CO₂ gas sensor signal (R_{g}/R₀), FIG. 3B is a graph showing evaluation results of sensitivity α, FIG. 3C is a graph showing evaluation results of changes of sensor resistance values (value after durability test/initial value).
FIG. 4 shows results of sensor signal (R_{g}/R₀) of a sensor comprising a gas sensing layer composed of Sm₂O₃ to four gases CO₂, H₂, CO and ethanol tested under the conditions of 20°C, 50% RH and an operating temperature of 300°C.
FIG. 5 shows results of sensor signal (R_{g}/R₀) of a sensor comprising a gas sensing layer composed of Eu₂O₃ to four gases CO₂, H₂, CO and ethanol tested under the conditions of 20°C, 50% RH and an operating temperature of 300°C.
FIG. 6 shows results of sensor signal (R_{g}/R₀) of a sensor comprising a gas sensing layer composed of Gd₂O₃ to four gases CO₂, H₂, CO and ethanol investigated under the conditions of 20°C, 50% RH and an operating temperature of 300°C.
FIG. 7 shows results of sensor signal (R_{g}/R₀) of a sensor comprising a gas sensing layer composed of Dy₂O₃ to four gases CO₂, H₂, CO and ethanol tested under the conditions of 20°C, 50% RH and an operating temperature of 300°C.
FIG. 8 shows results of sensor signal (R_{g}/R₀) of a sensor comprising a gas sensing layer composed of Er₂O₃ to four gases CO₂, H₂, CO and ethanol tested under the conditions of 20°C, 50% RH and an operating temperature of 300°C.
FIGS. 9A to 9C show comparison results of gas sensor signal (R_{g}/R₀) of five sensors each comprising one of gas sensing layers composed of Sm₂O₃, Eu₂O₃, Gd₂O₃, Dy₂O₃ and Er₂O₃, respectively, to four gases CO₂, H₂, CO and ethanol under the conditions of 20°C and 50% RH. FIG. 9A, FIG. 9B and FIG. 9C are graphs showing results of sensor signal evaluated under the conditions of operating temperatures of 250°C, 300°C and 350°C, respectively.
FIG. 10 is a graph showing comparison results of sensitivity α for five sensors each comprising one of gas sensing layers composed of Sm₂O₃, Eu₂O₃, Gd₂O₃, Dy₂O₃ and Er₂O₃, respectively, under the conditions of 20°C, 50% RH, and operating temperatures of 250°C, 300°C and 350°C.
FIG. 11 is a graph showing results of comparing CO₂ gas sensor signal (R_{g}/R₀) of sensors each comprising one of gas sensing layers composed of commercially available Gd₂O₃ and Dy₂O₃, respectively, and sensors, each comprising one of gas sensing layers composed of Gd₂O₃ and Dy₂O₃, respectively, produced in the present invention, under the conditions of carbon dioxide at a concentration of 1000 ppm at 20°C and 50% RH, and a sensor operating temperature of 300°C.

### DESCRIPTION OF PREFERRED EMBODIMENTS

Hereinafter, the embodiments of the present invention will be described with reference to the drawings. However, the present invention is not limited to the embodiments described below.

### [First embodiment: gas sensor]

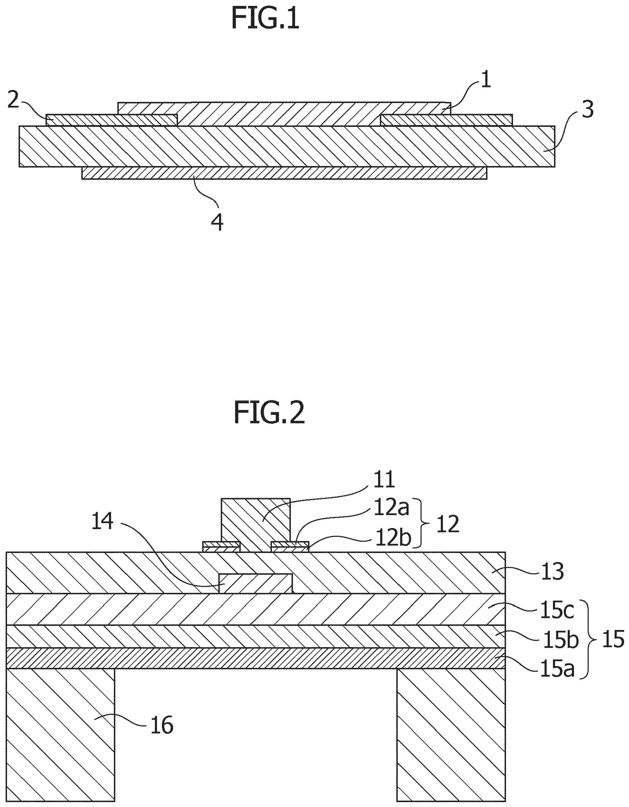

According to the first embodiment, the present invention relates to a CO₂ gas sensor. FIG. 1 is a schematic cross-sectional view showing one example of the gas sensor according to the first aspect of the present embodiment. Referring to FIG. 1, the gas sensor mainly comprises the gas sensing layer 1, electrodes 2, insulating substrate 3 and heating layer 4. FIG. 1 schematically shows a configuration of the gas sensor. The size and thickness of each part are not exact, and relative relationships of position and size are not limited to the aspects shown in the figure.

The insulating substrate 3 may be any substrate as long as it can ensure electrical insulation between the heating layer 4 and the electrodes 2. For example, a silicon substrate with the oxide film and an alumina substrate can be used, but the insulating substrate 3 is not limited thereto. The heating layer 4 is provided on one major surface of the insulating substrate 3. The heating layer 4 may be any layer as long as it can heat gas sensing layer 1 to a predetermined operating temperature through the insulating substrate 3. Pt film etc. can be used as the heating layer, but the heating layer 4 is not limited thereto. In the illustrated aspect, a gas sensor provided with a heating layer is exemplified; however, a heating layer may not be an essential constituent of the gas sensor of the present invention. A heating layer or an alternative heating device will be described below.

The electrodes 2 are provided on the major surface of the insulating substrate 3 opposite to the heating layer 4. The electrodes 2 are preferably a platinum (Pt) film or a gold (Au) film, and usually, comb teeth-shaped electrodes can be used.

The gas sensing layer 1 is provided on a major surface of the insulating substrate 3 so as to cover the electrodes 2. The gas sensing layer 1 comprises a chemoresistive material, and may optionally comprises an inorganic binder, aggregate, and conductive material etc. In the present invention, the chemoresistive material is a rare earth oxide. The rare earth oxide is one or more compounds selected from rare earth oxides represented by Ln₂O₃. In the chemical formula, Ln is selected from Sm (samarium), Eu (europium), Tb (terbium), Dy (dysprosium), Er (erbium), Yb (ytterbium). The rare earth oxide may be a composite metal oxide, which may comprise two or more metals selected from the above in any proportion. Among these, Sm₂O₃, Eu₂O₃, Dy₂O₃, Er₂O₃ and Yb₂O₃. are particularly preferable in terms of sensor signal and stability.

In the present invention, a main component of the rare earth oxide as a chemoresistive material is a rare earth oxide having a cubic crystal structure. "A main component of the rare earth oxide is rare earth oxide having a cubic crystal structure" means that at least 80 %, preferably 90 % of the rare earth oxide has cubic crystal structure, preferably the rare earth oxide substantially consists of rare earth oxide having a cubic crystal structure, and further preferably 100 % of the rare earth oxide is rare earth oxide having a cubic crystal structure. The content (%) of the rare earth oxide having a cubic crystal structure in the rare earth oxide can be calculated by measuring the ratio of peaks using an X-ray diffractometer.

In the present invention, gas sensing layer 1 does not comprise a semiconductor such as SnO₂ as a chemoresistive material, and the rare earth oxide is used alone as a chemoresistive material. When the SnO₂ is not comprised, an advantage of being able to enhance selectivity against interfering components such as H₂, CO and ethanol can be obtained.

Examples of an optional component of the gas sensing layer 1 include a binder and an aggregate for maintaining mechanical strength of the gas sensing layer 1. As a binder and an aggregate, those which are usually used can be used within the range not inhibiting chemoresistivity of the rare earth oxide, and for example, inorganic binders such as alumina sol can be exemplified but they are not limited to a specific material. Examples of other optional components include a conductive material for adjusting the resistivity of the gas sensing layer 1. These optional components may be included in an amount of 20 mass % or less, preferably 15 mass% or less, relative to the total mass of the gas sensing layer 1.

The heating layer 4 of the gas sensor is electrically connected to a driving processor, which is not shown, and the driving processor drives the heating layer 4. The gas sensing layer 1 is electrically connected to a driving processor, which is also not shown, via the electrodes 2 of the gas sensor, and the driving processor can read an electrical resistance value (referred to a sensor resistance value) of gas sensing layer 1. In the present embodiment, as a heating device for heating the gas sensing layer to a predetermined temperature, the heating layer provided on the side of the insulating substrate opposite to the gas sensing layer is illustrated. However, in the present invention, the shape of the heating device is not limited to a heating layer, and the arrangement of the heating device is also not limited to the aspect shown in the figure. In one embodiment, the heating device may be provided on the same surface of the insulating substrate as the gas sensing layer with the heating device being separated from the gas sensing layer. In another embodiment, the heating device may be provided on the major surface of the insulating substrate opposite to the surface on which the gas sensing layer is provided, and the heating device may be provided so as to be partially or completely embedded. In another embodiment, the heating device may be provided according to an aspect in which the heating device does not come into contact with the stack of the insulating substrate and the gas sensing layer, and for example, the heating device may be provided in a housing which contains the insulating substrate and the gas sensing layer. In any case, the heating device may be a heating layer or a heater which is not in the form of a layer, and may include one or more heating device, as long as the heating device can heat the gas sensing layer to a predetermined temperature.

Next, the gas sensor according to the present embodiment will be described with reference to a method of production. The method of production of the gas sensor according to the present embodiment comprises a step of forming the gas sensing layer 1 comprising the rare earth oxide illustrated above.

In producing the gas sensor, the heating layer 4 is formed on one major surface of the insulating substrate 3, and the electrodes 2 are formed on the other major surface. The heating layer 4 and the electrodes 2 on the insulating substrate 3 can be formed by a commonly used method. The heating layer 4 and the electrodes 2 can be respectively connected to a driving processor, which is not shown, by a commonly used method. As for a sensor provided with a heating device other than a heating layer, the heating device can be attached to a suitable place by a commonly used method and connected to a driving power source etc.

Forming the gas sensing layer 1 comprises a step of preparing solid powder of one or more rare earth oxides selected from rare earth oxides represented by Ln₂O₃ (Ln is same as defined above) which are main components of the gas sensing layer 1, and a step of mixing one or more rare earth oxides and a solvent and, if necessary, an optional component such as a binder to form a film on the insulating substrate 3 on which the electrodes 2 are formed.

The rare earth oxide prepared before film formation comprises a rare earth oxide having a cubic crystal structure as a main component, and preferably may comprise 100% of rare earth oxide having a cubic crystal structure.

In the step of mixing one or more rare earth oxides and a solvent, solvents which have high boiling point and lower volatility such as propane-1,2-diol, ethyl carbitol, diethylene glycol monoethyl ether, and ethylene glycol can be used. The rare earth oxide and the solvent are mixed thoroughly to obtain a paste, then a film is formed by a screen printing method, drop coating method, spray coating method etc. at a desired thickness on the insulating substrate 3 on which the electrodes 2 are formed. Then, the obtained film is dried at 60 to 80°C for 10 to 15 hours. After drying, the film is preferably heat-treated for 10 to 15 minutes under the heat treatment conditions identical to those for producing the rare earth oxide. Thus, the gas sensor can be obtained in which the heating layer 4 can be driven and electrical resistance values of the gas sensing layer can be read by electrifying the sensor.

As another aspect of the gas sensor according to the present embodiment, a diaphragm-type thin film gas sensor can be mentioned. FIG. 2 schematically shows a cross-section of a diaphragm-type thin film gas sensor. The diaphragm-type gas sensor comprises silicon substrate (hereinafter referred to as Si substrate) 16, thermally insulating support layer 15, heating layer 14, insulating substrate 13, electrodes 12, and gas sensing layer 11.

The Si substrate 16 is formed of silicon (Si), and through holes are formed on the Si substrate at the locations directly over which the gas sensing layer 11 is positioned. The thermally insulating support layer 15 covers the openings of the through holes to form a diaphragm, and is provided on the Si substrate 16. Specifically, the thermally insulating support layer 15 has a three-layer structure comprising thermally oxidized SiO₂ layer 15a, CVD-Si₃N₄ layer 15b and CVD-SiO₂ layer 15c. The thermally oxidized SiO₂ layer 15a is formed as a heat insulation layer, and has a function of reducing heat capacity by preventing heat generated in the heating layer 14 from being conducted to the side of the Si substrate 16. Furthermore, this thermally oxidized SiO₂ layer 15a has high resistance to plasm etching, which facilitates formation of through holes on the Si substrate 16 by plasma etching. The CVD-Si₃N₄ layer 15b is formed on upper side of the thermally oxidized SiO₂ layer 15a. The CVD-SiO₂ layer 15c enhances adhesion to the heating layer 14, and in addition, ensures electrical insulation. SiO₂ layer formed by CVD (chemical vapor deposition method) has a low internal stress.

The heating layer 14 may be a Pt-W film in the form of thin film, and is provided on the upper side of approximately the center of the thermally insulating support layer 15. Furthermore, the heating layer 14 is connected to a driving processor, which is not shown, and is configured to be subjected to power feeding. The insulating substrate 13 may be a sputtered SiO₂ layer for ensuring electrical insulation, and is provided so as to cover the thermally insulating support layer 15 and the heating layer 14. The insulating substrate 13 can ensure electrical insulation between the heating layer 14 and the electrodes 12a. Furthermore, the insulating substrate 13 can enhance adhesion to the gas sensing layer 11.

The bonding layer 12b is, for example, Ta film (tantalum film) or Ti film (titanium film), and a left-and-right pair of the bonding layers 12b is provided on the insulating substrate 13. These bonding layers 12b are interposed between the electrodes 12a and the insulating substrate 13 to enhance bonding strength. The electrodes 12a are, for example, Pt film (platinum film) or Au film (gold film), and a left-and-right pair of the electrodes 12a is provided so as to serve as sensing electrodes of the gas sensing layer 11. The gas sensing layer 11 is formed astride a pair of the electrodes 12a on the insulating substrate 13 across. In particular, the composition of the gas sensing layer 11 is the same as described in the embodiment with reference to FIG. 1. Specifically, the gas sensing layer 11 comprises one or more rare earth oxides selected from rare earth oxides (Ln is the same as defined above) represented by Ln₂O₃, and the rare earth oxide comprises rare earth oxide having a cubic crystal structure as a main component.

Similarly to the sensor according to the first aspect, the heating layer 14 of the gas sensor is electrically connected to a driving processor, which is not shown in the figures, and the driving processor drives the heating layer 14. Furthermore, the gas sensing layer 11 is electrically connected to a driving processor, which is also not shown, via the electrodes 12a of the gas sensor so that the driving processor can read electrical resistance values of the gas sensing layer 11.

A diaphragm-type gas sensor can be also obtained by forming a sensing layer using a specific rare earth oxide by the method described above to produce the gas sensor having the structure shown in FIG. 2. The material for film forming is the same as described above for the sensor shown in FIG. 1.

Such gas sensors having a diaphragm structure may provide high thermal insulation and low heat capacity. Furthermore, in the gas sensor, heat capacity of each constituent of electrodes 12a, gas sensing layer 11 and heating layer 14 can be reduced by techniques such as MEMS (micro-electrical-mechanical system). Therefore, temperature change over time is greater during driving of the heater, and thus, thermodesorption can be achieved in an extremely short time.

In the present embodiment, the gas sensor is described by showing specific examples of structures of the sensors in FIGS. 1 and 2. However, the present invention is not limited thereto, and the gas sensor may have any structure as long as the sensor comprises the structure in which the gas sensing layer is driven to be a predetermined temperature by a heating device and electrical resistance values of the gas sensing layer can be read. When the gas sensing layer described in the present embodiment is used, a compact high-performance CO₂ gas sensor having high stability can be provided.

### Producing Method for Rare Earth Oxide]

A method of producing a rare earth oxide is described. This method is not part of the present invention.

The method of producing a rare earth oxide (Ln is the same as defined above) represented by Ln₂O₃ comprises a step of heating the rare earth metal carboxylate or the rare earth metal carbonate, or the hydrate thereof at 425 to 575°C for 2 to 80 hours in a gas atmosphere.

In the production method, the rare earth metal carboxylate or the rare earth metal carbonate, or the hydrate thereof can be used as a starting material. As a rare earth metal constituting a rare earth metal carboxylate, those corresponding to Ln in the target rare earth oxide represented by Ln₂O₃ can be used, and the rare earth metal can be selected from Ln defined above. Specific examples of rare earth metal carboxylates include, but are not limited to, oxalates represented by Ln₂[C₂O₄]₃ or oxalate hydrates represented by Ln₂[C₂O₄]₃•nH₂O, carbonates represented by Ln₂[CO₃]₃ or hydrate thereof, acetates represented by Ln[CH₃COO]₃ or hydrates thereof.

In a step of heating, a rare earth metal carboxylate or a rare earth metal carbonate, or a hydrate thereof which is in the form of solid powder at room temperature can be preferably placed in a heat resistant open-type alumina container and the like, and heated in a heating furnace. The heating temperature is preferably 425 to 575°C, and this is preferably maintained at a constant temperature during heating. The heating time may be 2 to 80 hours. The atmosphere during heating is not particularly limited, but it may be air, a closed system, or an atmosphere to which gas such as air can be continuously supplied. As one example of an atmosphere to which gas such as air can be continuously supplied, the atmosphere may be used in which gas comprising 350 to 500 ppm of carbon dioxide and moisture of 20 to 80% relative humidity at 20°C can be supplied. However, supplying a gas including carbon dioxide and moisture is not essential.

Among rare earth oxides, the heating conditions in producing Nd₂O₃ is preferably at 525 to 575°C for about 2 to 80 hours, or at 475 to 525°C for about 50 to 80 hours. The heating conditions in producing Sm₂O₃ is preferably at 525 to 575°C for about 2 to 80 hours, or at 475 to 525°C for about 15 to 80 hours, or at 425 to 475°C for about 60 to 80 hours. For all rare earth oxides, producing under the conditions of heating at high temperature for a long time is preferable in terms of heat stability, for example, heating at 525 to 575°C for 50 to 80 hours is preferable.

According to the production method of the rare earth oxide, a rare earth oxide comprising rare earth oxide having a cubic crystal structure as a main component can be produced. Such a rare earth oxide can be used for a chemoresistive material for a gas sensor, especially for a constituent of a gas sensing layer. The gas sensor comprising the rare earth oxide produced according to the present method as a constituent of the gas sensing layer is also useful as a detection sensor of carbon dioxide gas. In particular, carbon dioxide gas can be selectively detected while being distinguished from various gases such as hydrogen gas, carbon monoxide gas ethanol.

### EXAMPLES

Hereinafter, the present invention will be described in more detail with reference to the following examples. However, the present invention is not limited to the scope of the following Examples.

### (1) Producing and Evaluation of Rare Earth Oxide

Commercially available solid powder of Ln₂[C₂O₄]₃•nH₂O (Ln is Ce, Gd, Er, all produced by Sigma-Aldrich Co. LLC) or Ln(C₂H₃O₂)₃•nH₂O(Ln is Nd, Sm, Eu, Dy, Yb, all produced by Sigma-Aldrich Co. LLC) was used as a starting material. The powder of the starting material was placed in an alumina container and heated using a heating furnace. Air was continuously supplied to the heating furnace by a pump during heating. After the specified heating treatment, the obtained product was subjected to crystal structure analysis using an X-ray diffractometer. The starting materials, heat treatment conditions and XRD results of the products after heat treatment are shown in Table 1.

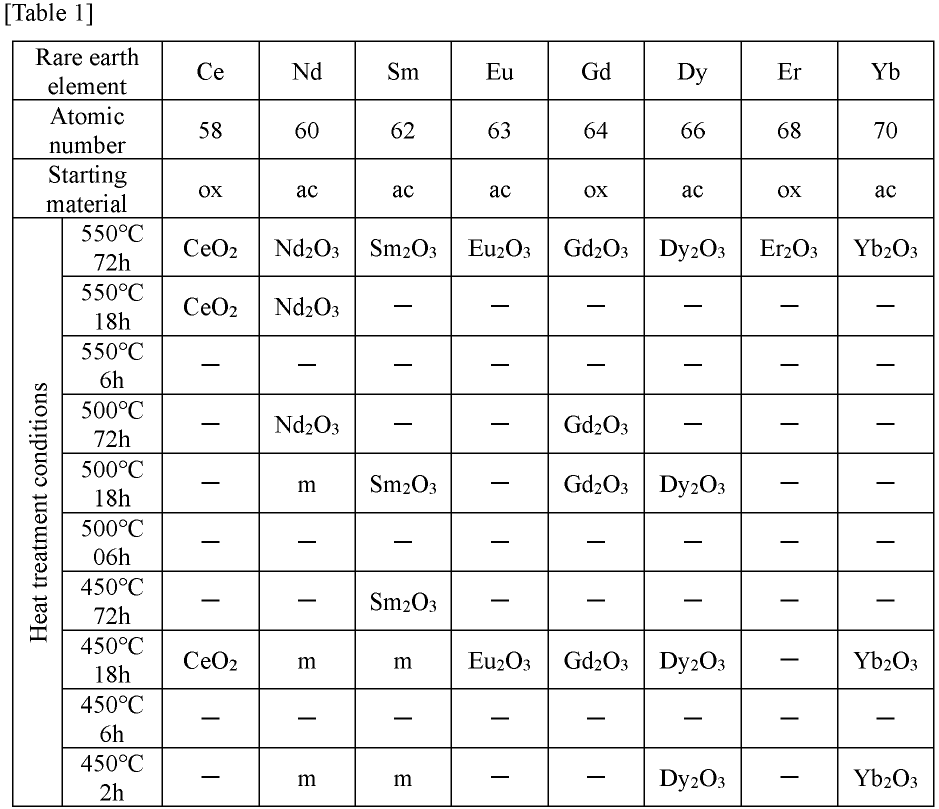

In table 1, "ox" means an oxalate, "ac" means an acetate. An "m" means that a monoclinic oxycarbonate was produced. A "-" means that a heating experiment was not conducted in the corresponding conditions.

The crystal structures of the rare earth oxides produced in the respective conditions shown in Table 1 were investigated by X-ray crystallographic diffraction, and thus, the results shown in Table 2 below were obtained for crystal structures of the rare earth oxides which had been obtained by heat treatment at 550°C for 72 hours. In Table 2, "cubic" means a cubic crystal structure, "hexagonal" means a hexagonal crystal structure, and "cubic + hexagonal" means a state in which a cubic crystal structure and a hexagonal crystal structure were mixed. The crystal structure of the rare earth oxides used as a component of a sensing layer for producing the gas sensor in Example (2) described below was investigated by X-ray crystallographic diffraction after evaluation of the gas sensor properties of Example (3). As a result, no changes in crystal structures were observed for any of the oxides.

**[Table 2]**

| Oxide | Crystal structure (by XRD) |
|---|---|
| CeO₂ | cubic |
| Nd₂O₃ | cubic + hexagonal |
| Sm₂O₃ | cubic |
| Eu₂O₃ | cubic |
| Gd₂O₃ | cubic |
| Dy₂O₃ | cubic |
| Er₂O₃ | cubic |
| Yb₂O₃ | cubic |

### (2) Production of gas sensor

Among the oxides produced in above (1), eight rare earth oxides obtained by heat treatment at 550°C for 72 hours were used to produce the gas sensors. Specifically, the gas sensor shown in FIG. 1 was produced. An alumina substrate having thickness of 900 µm was used as the insulating substrate 3, and a Pt heater having thickness of 5 µm was provided on one major surface of the insulating substrate 3. A comb teeth-shaped Pt film having thickness of 5 µm was used as the electrodes 2, and the gap between the teeth of a comb was 10 µm. The solid powder of the oxide in Table 1 produced in (1) and propane-1,2-diol were mixed by a vibration mill at 30 Hz for 30 minutes, then the obtained paste was screen-printed on the insulating substrate 3 provided with the Pt electrodes 2, and thus, the gas sensing layer 1 was produced. The thickness of the gas sensing layer 1 as measured from the surface of the insulating substrate 3 was 50 µm.

The Pt heater was connected to a DC power source, which is not shown, and thus the sensor was enabled to be heated to a temperature of 250°C, 300°C or 350°C. The gas sensing layer 1 was connected to an electrical resistance measurement apparatus which is not shown via the electrodes 2 to provide a configuration which enabled measurement of DC resistance of the gas sensing layer at 10 second intervals.

### (3) Evaluation results

### (3-1) CO₂ sensor signal, sensitivity and durability

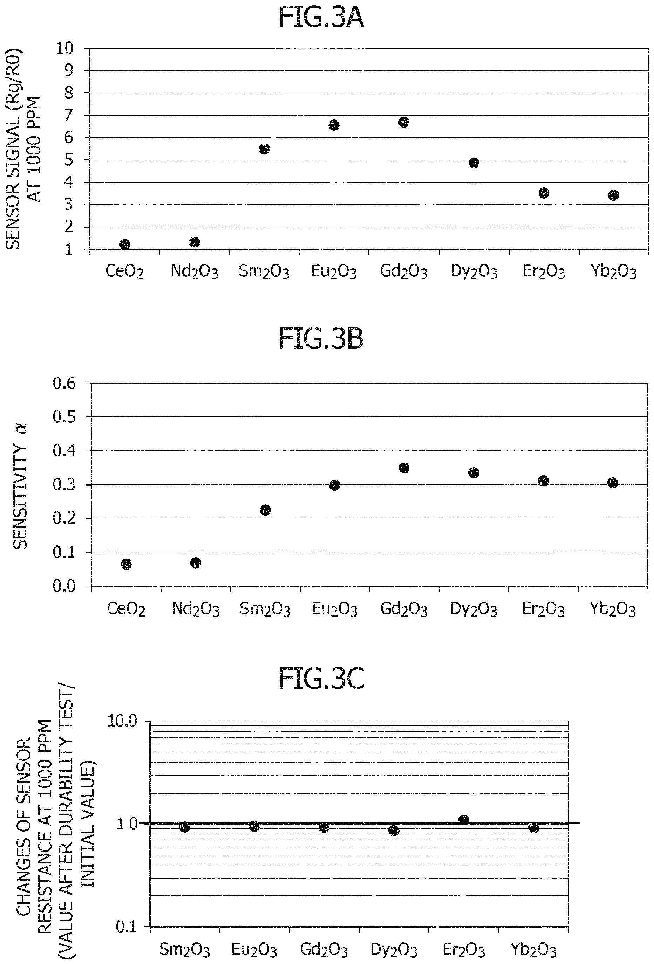

CO₂ sensor signal and sensitivity of the eight rare earth oxides shown in Table 1 were compared. FIG. 3A shows CO₂ gas sensor signal (R_{g}/R₀), FIG. 3B shows sensitivity α. Herein, the CO₂ gas sensor signal R_{g}/R₀ represents (DC resistance value of the sensor when the sensor is driven at a specified CO₂ concentration)/(DC resistance value of the sensor when the sensor is driven at CO₂ concentration of 0 ppm). Measurements of gas sensor signal were conducted at CO₂ concentration of 1000 ppm, at 20°C, 50 % RH, a sensor operating temperature of 300°C. Sensitivity α is an indicator defined as R_{g}/R₀ = A×[CO₂ concentration]^{α}, wherein A and α are constant numbers. In FIG. 3A and 3B, the horizontal axis follows the order of atomic number of rare earth elements. Both CO₂ sensor signal and sensitivity α were maximum around Gd.

FIG. 3C shows results of durability test conducted for six oxides: Sm₂O₃, Eu₂O₃, Gd₂O₃, Dy₂O₃, Er₂O₃ and Yb₂O₃. The durability test was conducted by operating the sensor for 3 days in an atmosphere with a high CO₂ concentration and high humidity (3000 ppm, 20°C, 80 % RH) at an operating temperature of the gas sensor of 350°C which was higher than the standard temperature of 300°C, and evaluating gas sensor signal (R_{g}/R₀) before and after electrifying. The gas sensor signal measurement was conducted before and after the durability test, at CO₂ concentration of 1000 ppm, 20°C, 50 % RH and an operating temperature of 300°C. The changes of sensor resistivity values before and after the durability test were about 1 for all rare earth oxides, which shows the gas sensor has high durability. Although not shown in the figure, both gas sensor signal and sensitivity were stable even after the durability test.

### (3-2) Selectivity and CO₂ sensitivity over range up to high concentration

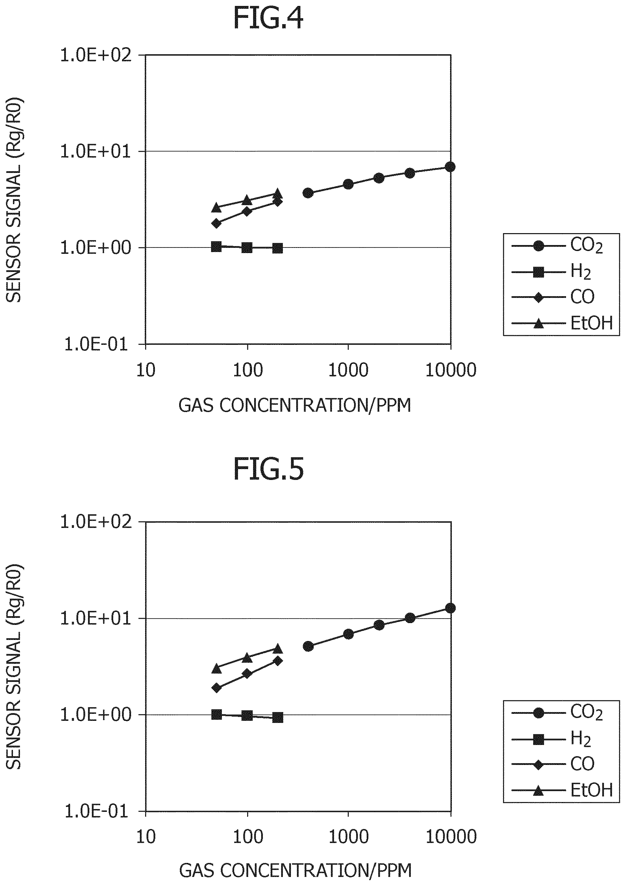

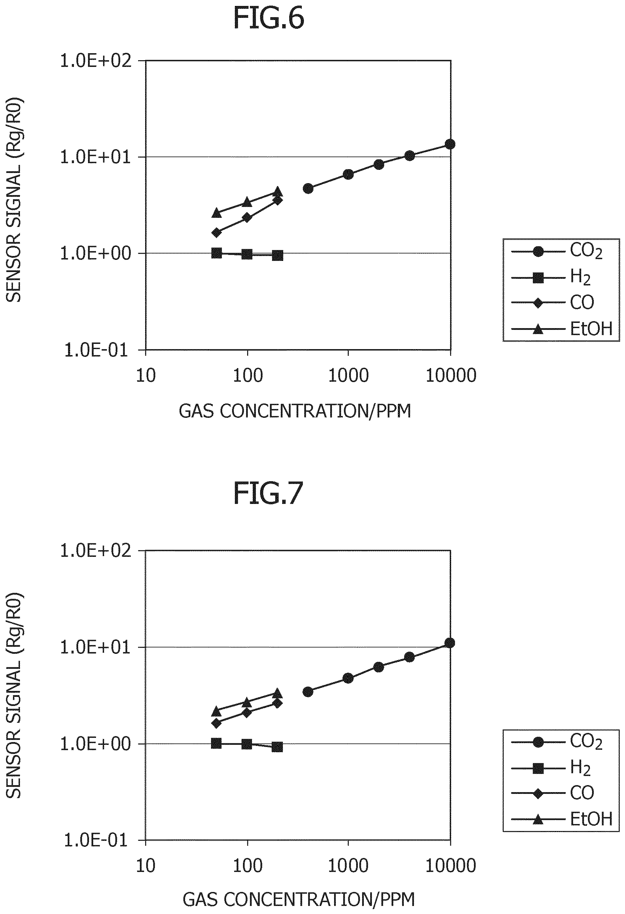

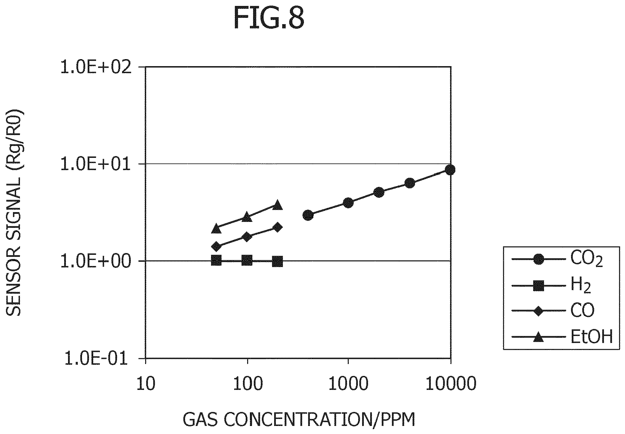

FIGS. 4 to 8 show results of measuring sensor signal to four gases CO₂, H₂, CO and ethanol for five rare earth oxides Sm₂O₃, Eu₂O₃, Gd₂O₃, Dy₂O₃ and Er₂O₃ and evaluating gas selectivity and CO₂ sensitivity over the range up to high concentration. A gas sensor signal R_{g}/R₀ represents (DC resistance value of the sensor when the sensor is driven at a specified gas concentration)/ (DC resistance value of the sensor when the sensor is driven in an atmosphere not comprising CO₂). The measurement of gas sensor signal was conducted under the conditions of 20°C, 50 % RH and a sensor operating temperature of 300°C. For all oxides, CO₂ sensor signal were linear over the range up to 10,000 ppm in double logarithmic graphs and sensitivity were almost the same.

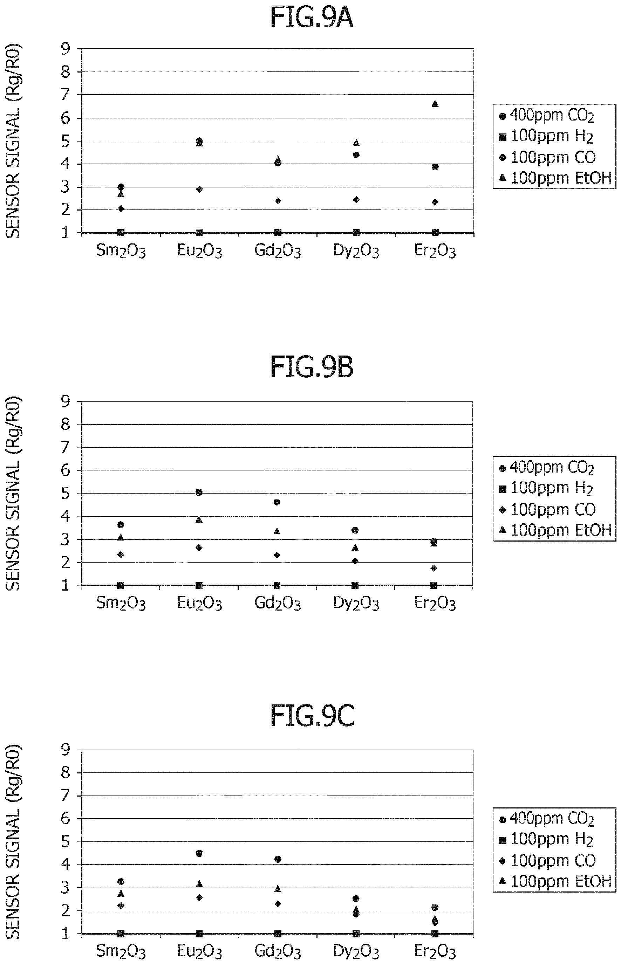

FIGS. 9A to 9C are graphs showing sensor signal to 400 ppm of CO₂ and 100 ppm of hydrogen, CO and ethanol (Et-OH) which were extracted from data of FIGS. 4 to 8. The measurement conditions of gas sensor signal were at 20°C and 50 % RH in FIGS. 9A to 9C, and FIG. 9A shows gas sensor signal when the operating temperature of the gas sensor was 250°C, FIG. 9B shows gas sensor signal when the operating temperature was 300°C and FIG. 9C shows gas sensor signal when the operating temperature was 350°C. A CO₂ concentration of 400 ppm is the lowest concentration within the range estimated for the current atmospheric environment level. On the other hand, hydrogen, CO and ethanol concentrations of 100 ppm were at highest concentrations within the range estimated very severely. For all sensors using the rare earth oxides, when the operating temperature was 300°C or 350°C, CO₂ sensor signal was not lower than sensor signal to other various gases. Among various gases, sensor signal to ethanol and CO were high, in this order, and sensor signal to hydrogen was almost zero.

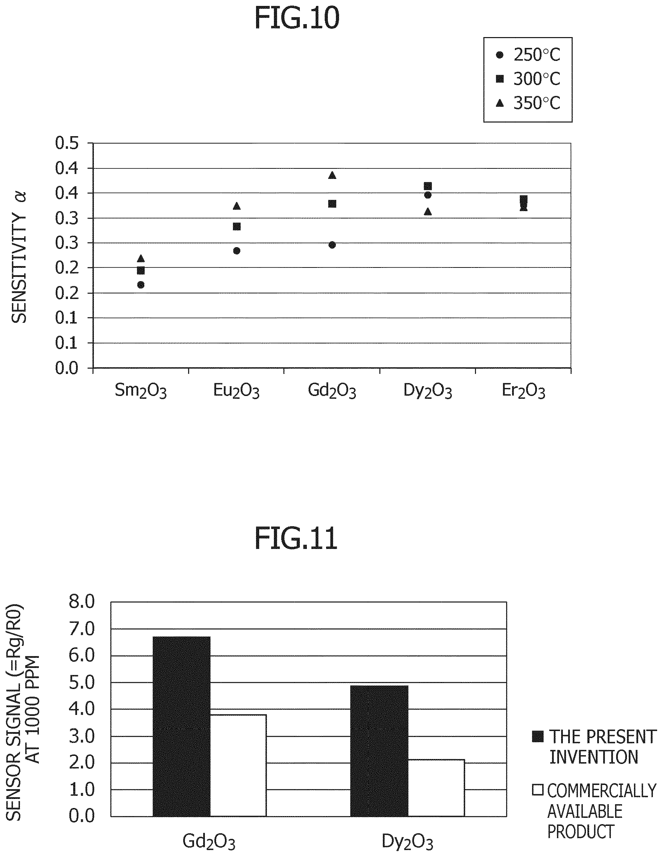

FIG. 10 shows results of comparing carbon dioxide sensitivity α (average value between 400 ppm and 10,000 ppm) of the sensors comprising the rare earth oxide as a sensing layer at an operating temperature of 250°C to 350°C. The sensitivity α was highest at Gd or Dy, and the sensitivity α increased with the temperature at Gd or lighter elements. On the other hand, results in reverse order were obtained at Dy or a heavier element. It was suggested that when the optimum combination of a rare earth oxide and an operating temperature is selected, the selectivity and sensitivity can be adjusted to the various required levels, depending on application.

### (3-3) Comparison with commercially available product with respect to CO₂ sensor signal

Next, the CO₂ sensor signal of Gd₂O₃ and Dy₂O₃ each having a cubic crystal structure produced in Example (1) and obtained by heat treatment at 50°C and 72 hours were compared to the CO₂ sensor signal of commercially available Gd₂O₃ and Dy₂O₃ (Sigma-Aldrich Co. LLC, product number 637335, 637289, specification of particle size <100 nm). Measurements of CO₂ gas sensor signal (R_{g}/R₀) were conducted at CO₂ concentration of 1000 ppm, 20°C, 50% RH and a sensor operating temperature of 300°C similarly to the above (3-1). The comparison results of CO₂ sensor signal is shown in FIG. 11. Even in the case of using the commercially available rare earth oxide for a sensing layer, sensor signal to CO₂ could be obtained, but in the case of using the rare earth oxide produced by the method of Example (1) for a sensing layer, sensor signal of about 2 to 2.5 times as high as that of the commercially available product could be obtained. From this result, it was confirmed that rare earth oxide more suitable for a gas sensor can be obtained by the method of Example (1).

### INDUSTRIAL APPLICABILITY

The gas sensor according to the present invention is useful as a MEMS solid-state gas sensor with low power consumption, which is intended to be battery-driven.

### REFERENCE SIGNS LIST

1 Gas sensing layer
2 Electrode
3 Insulating substrate
4 Heating layer
11 Gas sensing layer
12a Electrode
12b Bonding layer
13 Insulating substrate
14 Heating layer
15 Thermally insulating support layer
16 Si substrate

## Claims

1. A carbon dioxide gas sensor comprising
an insulating substrate (13), electrodes (12) and a gas sensing layer (11) formed on one major surface of the insulating substrate (13) so as to cover the electrodes,
wherein the gas sensing layer (11) comprises one or more compounds selected from rare earth oxides represented by Ln₂O₃, and
at least 80 % of the rare earth oxide has a cubic crystal structure,
wherein the rare earth oxide represented by Ln₂O₃ is at least one of Sm₂O₃, Eu₂O₃, Tb₂O₃, Dy₂O₃, Er₂O₃ and Yb₂O₃.

2. A method for producing a carbon dioxide gas sensor comprising a step of
forming an insulating substrate (13), electrodes (12) and a gas sensing layer (11) formed on one major surface of the insulating substrate (13) so as to cover the electrodes, wherein the gas sensing layer comprises at least one compound selected from rare earth oxides represented by Ln₂O₃, and at least 80 % of the rare earth oxide has a cubic crystal structure as a main component, wherein the rare earth oxide represented by Ln₂O₃ is at least one of Sm₂O₃, Eu₂O₃, Tb₂O₃, Dy₂O₃, Er₂O₃ and Yb₂O₃.

## Patentansprüche

1. Kohlendioxidgassensor, umfassend
ein Isoliersubstrat (13), Elektroden (12) und eine Gaserfassungsschicht (11), die auf einer Hauptfläche des Isoliersubstrats (13) gebildet ist, sodass die Elektroden abdeckt sind,
wobei die Gaserfassungsschicht (11) eine oder mehrere Verbindungen umfasst, die aus durch Ln₂O₃ repräsentierten Seltenerdoxiden ausgewählt sind, und
mindestens 80 % des Seltenerdoxids eine kubische Kristallstruktur aufweist,
wobei das durch Ln₂O₃ repräsentierte Seltenerdoxid mindestens eines von Sm₂O₃, Eu₂O₃, Tb₂O₃, Dy₂O₃, Er₂O₃ und Yb₂O₃ ist.

2. Verfahren zur Herstellung eines Kohlendioxidgassensors, umfassend einen Schritt des Bildens eines Isoliersubstrats (13), von Elektroden (12) und einer Gaserfassungsschicht (11), die auf einer Hauptfläche des Isoliersubstrats (13) gebildet wird, sodass die Elektroden abdeckt sind, wobei die Gaserfassungsschicht mindestens eine Verbindung umfasst, die aus durch Ln₂O₃ repräsentierten Seltenerdoxiden ausgewählt ist und mindestens 80 % des Seltenerdoxids eine kubische Kristallstruktur als Hauptkomponente aufweist, wobei das durch Ln₂O₃ repräsentierte Seltenerdoxid mindestens eines von Sm₂O₃, Eu₂O₃, Tb₂O₃, Dy₂O₃, Er₂O₃ und Yb₂O₃ ist.

## Revendications

1. Capteur de dioxyde de carbone gazeux comprenant
un substrat isolant (13), des électrodes (12) et une couche de détection de gaz (11) formée sur une surface majeure du substrat isolant (13) de façon à couvrir les électrodes,
dans lequel la couche de détection de gaz (11) comprend un ou plusieurs composés choisis parmi les oxydes des terres rares représentés par Ln₂O₃, et
au moins 80 % de l'oxyde des terres rares ont une structure cristalline cubique,
dans lequel l'oxyde des terres rares représenté par Ln₂O₃ est au moins l'un parmi Sm₂O₃, Eu₂O₃, Tb₂O₃, Dy₂O₃, Er₂O₃ et Yb₂O₃.

2. Procédé pour produire un capteur de dioxyde de carbone gazeux, comprenant une étape de
formation d'un substrat isolant (13), d'électrodes (12) et d'une couche de détection de gaz (11) formée sur une surface majeure du substrat isolant (13) de façon à couvrir les électrodes,
dans lequel la couche de détection de gaz comprend au moins un composé choisi parmi les oxydes des terres rares représentés par Ln₂O₃, et au moins 80 % de l'oxyde des terres rares ont une structure cristalline cubique en tant que composant principal, dans lequel l'oxyde des terres rares représenté par Ln₂O₃ est au moins l'un parmi Sm₂O₃, Eu₂O₃, Tb₂O₃, Dy₂O₃, Er₂O₃ et Yb₂O₃.
